# EUROPEAN PATENT SPECIFICATION

(11) **EP 3 386 558 B1**
(45) Date of publication and mention of the grant of the patent: **04.10.2023**
(21) Application number: 16822313.9
(22) Date of filing: 09.12.2016
(51) Int. Cl.: A61L 31/14, A61L 2/08, A61L 31/04

(54) **STERILISATION METHODS FOR A POLY(ESTER UREA) FIBER MATERIAL**
POLY(ESTERHARNSTOFF)-STERILISATIONSVERFAHREN
PROCÉDÉS DE STÉRILISATION DE FIBRES DE POLY(ESTER URÉE)

(30) Priority: 10.12.2015 US 201562265517 P
(43) Date of publication of application: 17.10.2018
(73) Proprietor: Cook Biotech Incorporated, West Lafayette, IN 47906 (US)
(72) Inventor: WADE, Mary Beth, Dublin, Ohio 43016 (US); PATEL, Umesh H., West Lafayette, IN 47906 (US); SHAH, Bhavin, West Lafayette, IN 47906 (US)
(74) Representative: Marks & Clerk LLP
(86) International application number: PCT/US2016/065899
(87) International publication number: WO 2017/100625

(56) References cited:
- WO-A1-2012/166594
- WO-A1-2015/048728
- US-A1- 2007 071 790
- US-A1- 2007 128 250
- US-A1- 2010 074 934
- US-A1- 2015 297 798
- ANGELICA DIAZ ET AL: "New poly(ester urea) derived from l-leucine: Electrospun scaffolds loaded with antibacterial drugs and enzymes", MATERIALS SCIENCE AND ENGINEERING C, vol. 46, 1 January 2015 (2015-01-01), pages 450-462, XP055577540, ISSN: 0928-4931, DOI: 10.1016/j.msec.2014.10.055
- Abraham G. A. ET AL: "Physical and mechanical behavior of sterilized biomedical segmented polyurethanes", Journal of Applied Polymer Science, vol. 65, no. 6, 8 August 1997 (1997-08-08) , pages 1193-1203, XP055881642, US ISSN: 0021-8995, DOI: 10.1002/(SICI)1097-4628(19970808)65:6<1193 ::AID-APP15>3.0.CO;2-V
- Iaea: "TECHNICAL REPORTS SERIES No. 149 - "Manual on Radiation Sterilization of Medical and Biological Materials"", , 1 January 1973 (1973-01-01), pages 1-343, XP055881764, Vienna, Austria Retrieved from the Internet: URL:https://inis.iaea.org/collection/NCLCo llectionStore/_Public/05/105/5105679.pdf [retrieved on 2022-01-20]
- Kimberly Sloan Stakleff ET AL: "Resorbable, amino acid-based poly(ester urea)s crosslinked with osteogenic growth peptide with enhanced mechanical properties and bioactivity", Acta Biomaterialia, vol. 9, no. 2, 1 February 2013 (2013-02-01), pages 5132-5142, XP055144368, ISSN: 1742-7061, DOI: 10.1016/j.actbio.2012.08.035
- Mary Beth Wade ET AL: "Influence of Sterilization Technologies on Electrospun Poly(ester urea)s for Soft Tissue Repair", Biomacromolecules, vol. 17, no. 10, 10 October 2016 (2016-10-10), pages 3363-3374, XP055767261, US ISSN: 1525-7797, DOI: 10.1021/acs.biomac.6b01158
- Mine Silindir ET AL: "Sterilization Methods and the Comparison of E-Beam Sterilization with Gamma Radiation Sterilization", FABAD Journal of Pharmaceutical Sciences, 1 March 2009 (2009-03-01), pages 43-53, XP055613021, Ankara Retrieved from the Internet: URL:http://dergi.fabad.org.tr/pdf/volum34/ issue1/43-53.pdf

## Description

### PRIORITY APPLICATION

The present application claims the right of priority to U.S. Provisional Patent Application Number 62/265,517 which was filed on December 10, 2015.

### BACKGROUND

Medical devices can be fabricated from many different materials. These materials may be selected for their properties, such as their biocompatibility, morphology, mechanical properties, and/or for other reasons. Because medical devices are typically implanted in a patient, these devices must be sterilized to reduce the probability of infection in the patient's body. Angelica Diaz Et Al: "New poly(ester urea) derived from l-leucine: Electrospun scaffolds loaded with antibacterial drugs and enzymes", Materials Science and Engineering C, vol. 46, 1 January 2015 (2015-01-01), pages 450-462, XP055577540, ISSN: 0928-4931, DOI: 10.1016/j.msec.2014.10.055 describes the synthesis of poly(ester urea) 1L6 electrospun scaffolds which are subjected to UV-radiation.

The probability of a unit being non-sterile after it has undergone a sterilization process is commonly called the Sterility Assurance Level (SAL) and is usually low, for example having a probability of 10⁻⁶. When SAL is used to describe the efficacy of a sterilization process, the inverse of SAL is typically used, for example, 10⁶. Different processes and different products may have different SALs.
There remain needs for sterilized medical devices and methods for sterilizing materials.

### SUMMARY

The present invention is defined by the appended claims.
Claimed is a method for sterilizing a material comprising poly(ester urea) polymer fibers, comprising the steps of:
exposing a material comprising poly(ester urea) polymer fibers to radiation to sterilize the material;
   wherein the material is an electrospun fiber material incorporating the poly(ester urea) polymer fibers and the maximum diameter of the fibres is 10 µm;
characterized in that said radiation is electron beam radiation at a dosage of between 5 kGy and 50 kGy and the electron beam has an energy level of 5 MeV to 20 MeV; and
the method produces a material comprising poly(ester urea) polymer fibers; and the material has a sterility assurance level with a probability level of less than about 10-6 after the step of exposing the material to radiation.
Poly(ester urea) fibers of the present invention are formed by electrospinning and may be used to construct medical devices, including, but not limited to hernia repair or other tissue support grafts. In some embodiments, the irradiation comprises an electron beam having an energy of about 10 megaelectronvolts (MeV). In other embodiments, irradiation by an electron beam is performed for greater than about 1 minute. In some embodiments, poly(ester urea) fibers of the present disclosure may be crosslinked after exposure to an electron beam. In other embodiments, poly(ester urea) fibers of the present disclosure are not crosslinked after exposure to an electron beam.

In other aspects, poly(ester urea) fibers of the present disclosure may have a number-average and/or weight-average molecular weight of greater than about 5,000 daltons (D) as determined by size exclusion chromatography after exposing a material comprising poly(ester urea) polymer fibers to irradiation by an electron beam.

In some embodiments, poly(ester urea) fibers may comprise the following chemical structure: 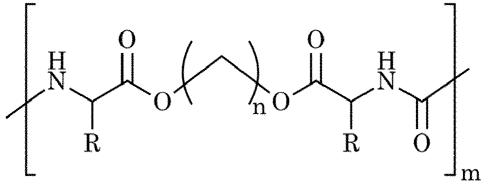 In certain embodiments, R is selected from any of the naturally occurring amino acid side-chains, and in some preferred embodiments, R is isobutyl and/or benzyl substituents.

In some further embodiments, poly(ester urea) fibers may be crosslinked. In certain embodiments, a peptide-based crosslinker may be used. In some preferred embodiments, the peptide based crosslinker has the following structure: 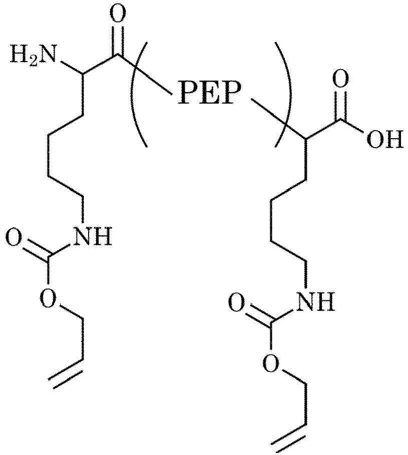 where PEP is a polypeptide chain comprising from 2 to 20 amino acid residues.

### BRIEF DESCRIPTION OF THE DRAWINGS

Fig. 1 shows the synthesis of an amino-acid-based poly(ester urea) polymer of one embodiment of the present disclosure.
Fig. 2 shows a digital image of a scanning electron micrograph (SEM) of poly(ester urea) fibers before and after sterilization by electron beam irradiation.
Fig. 3 shows a digital image of a SEM of poly(ester urea) fibers (not presently claimed per se) before and after sterilization by ethylene oxide.
Fig. 4 shows a top view of one embodiment of the present invention where poly(ester urea) fibers are formed into a hernia graft for repairing a hernia.

### DETAILED DESCRIPTION

For the purposes of promoting an understanding of the principles of the invention, reference will now be made to certain embodiments and specific language will be used to describe the same.

In certain embodiments, the poly(ester urea) polymers of the present disclosure may be thermoplastic polymers. In some embodiments, the poly(ester urea) polymers of the present disclosure may be thermoplastic polymers and/or not thermoset polymers after irradiation by an electron beam to sterilize the polymer.

Poly(ester urea) polymers are a class of bio-absorbable polymers comprising ester and urea functional groups. In some embodiments, poly(ester urea) polymers may comprise amino acid sub-units as well as di-, tri-, or polyhydroxyl sub-units. In certain embodiments, a poly(ester urea) polymer has the chemical structure: 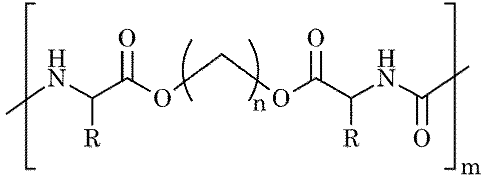 where 'n' and 'm' are independently selected positive integers greater or equal to 1. In some embodiments, 'n' may be a positive integer between 1 and 20. In other embodiments, 'n' may be 6, or 10. In certain embodiments, `m' is a positive integer between 1 and 1000.

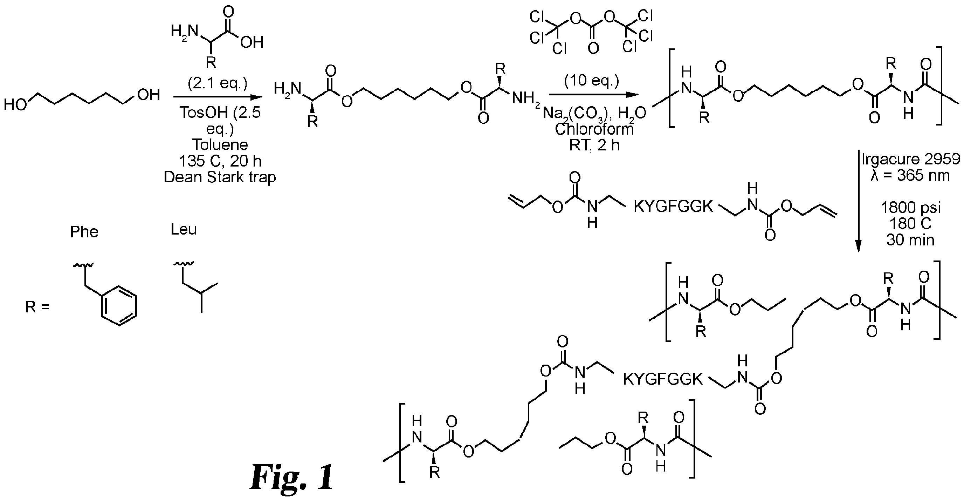

The present disclosure provides a particular reaction scheme that is suitable for forming the materials of this disclosure and is shown in Figure 1. A hydroxy-functionalized small molecule, typically either a diol or triol, can be reacted with an amino acid, which results in end-functionalizing the molecule with an amino acid based material, forming what is termed herein as an amino acid-functionalized monomer. A urea bond can then be introduced into the amino acid end-functionalized monomer using either triphosgene, diphosgene, or phosgene to form a poly(ester urea). The poly(ester urea) may be crosslinked with a peptide-based crosslinker to form the peptide-crosslinked amino acid-based poly(ester urea) material in some embodiments.

The hydroxy-functionalized small monomer may be selected from virtually any organic molecule of less than twenty carbons and having at least two hydroxy-end groups. In other embodiments, hydroxyl-functionalized compound may possess between 3 and 8 hydroxyl functional groups. These groups may arise from sugar molecules, carbohydrates, and/or linear or branched diols, triols, and/or polyols. In particular embodiments, the hydroxyl-functionalized small monomer may be 1,6-hexanediol; 1,8-octanediol; 1,10-decanediol, and/or 1,12-dodecanediol.

The amino acid may be selected from virtually any naturally occurring and/or non-naturally occurring amino acid, with the limitation that serine in its unprotected form may or may not be suitable because of the hydroxyl present on the side chain. However, suitably protected serine units may be employed in materials of the present disclosure if protection/deprotection steps are utilized as would normally occur to one of ordinary skill in the art if needed. In certain embodiments, phenylalaine (PHE) and/or leuceine (LEU) are preferred amino acids used in the preparation of materials of the present disclosure. In other embodiments, valine (VAL) and/or isoleucine (ILE) may be preferred amino acids used in the preparation of materials of the present disclosure.

The reaction of a hydroxyl-functionalized molecule with amino acid or amino acids to create an amino acid-functionalized monomer can be achieved in any number of ways generally known to those of skill in the art. Generally, a condensation reaction at temperatures exceeding the boiling point of water involving a slight molar excess (about 2.1 equivalents (eq.)) of the acid relative to the hydroxyl groups is sufficient to enable the reaction to proceed. The presence of toluene sulphonic acid may be necessary to protonate the amine on the amino acid and ensure that transamidation reactions do not occur at higher conversions.

A carbonyl synthon may be utilized to introduce a urea bond to the resultant amino acid-functionalized monomer. Typically, phosgene, diphosgene or triphosgene is employed. Diphosgene (a liquid) and/or triphosgene (a solid crystal) may be found more suitable than phosgene because they are generally appreciated as safer substitutes to phosgene, which is a toxic gas. However, other carbonyl synthons may be utilized as would normally occur to one of ordinary skill in the art.

The reaction of an amino acid functionalized monomer with triphosgene, diphosgene and/or phosgene to create an amino acid-based poly(ester urea) can also be achieved in any number of ways generally known to those of skill in the art. Generally, a large molar excess of a carbonyl synthon (about 10-50 molar excess relative to the amine concentration) may be used to drive the reaction to higher molecular masses.

In a specific embodiment, 1,6-hexanediol is employed. The diol may be reacted with 2.1 equivalents of an amino acid as shown in Figure 1, by mixing with toluene sulfonic acid (TsOH, 2.5 equivalents) and toluene at 135 degrees Celsius (°C) for 20 hours. An amino acid may react with a hydroxyl group of the diol to provide an amino acid-functionalized monomer as shown in Figure 1. Urea bonds are then introduced to this amino acid functionalized monomer by reaction with triphosgene, mixed with sodium carbonate, water and chloroform. This creates an amino acid-based poly(ester urea), as shown in Figure. 1.

In certain embodiments, the poly(ester urea) polymers of the present disclosure have a molecular weight of about 5,000 Daltons (D) to about 200,000 Daltons (D), more preferably about 40,000 Daltons (D) to about 200,000 Daltons (D).

In some embodiments, an amino acid-based poly(ester urea) may be crosslinked with a cross linker. In other embodiments, the amino acid-based poly(ester urea) is not crosslinked with a cross linker.

In some embodiments, the present disclosure provides peptide-crosslinked mechanically-robust and bioactive polymeric materials. In other embodiments, the present disclosure provides peptide-crosslinked amino acid-based poly(ester urea) materials that can be used for useful applications. In still other embodiments, the present disclosure provides peptide-crosslinked amino acid poly(ester urea) materials that provide osteoinductive activity. In other embodiments, the present disclosure provides a specific scaffold structure formed from peptide-crosslinked amino acid poly(ester urea) materials.

A peptide crosslinker may be employed to crosslink the amino acid-based poly(ester urea). Such a peptide crosslinker may be selected from those with the general structure indicated below:

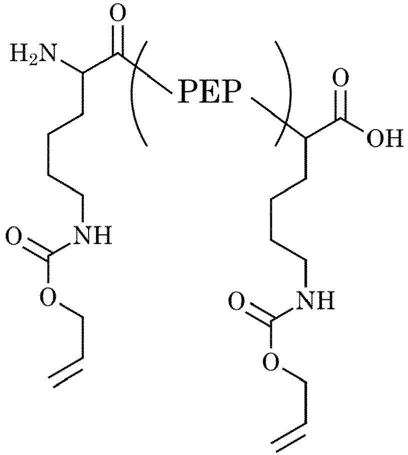

When such a crosslinker is employed, PEP may be selected from any peptide with 20 or less amino acids, and may have a desired bioactivity, preferably, PEP is a polypeptide chain comprising from 2 to 20 amino acid residues, and may have a desired bioactivity,. Such bioactivity includes, but is not limited to osteoconductivity, osteoinductivity, adhesive, anti-inflammatory, angiogenic, and/or neurostimulatory affects. In some embodiments, lysine may be employed at each end of the PEP, and the lysine residue may be used to form the allyl carbamate or urethane as shown in the above structure.

In particular embodiments, PEP may be chosen from bone sialoprotein (KRSR, sequence GGGKRSR, SEQ ID NO: 1), vitronectin, fibronectin (RGD, sequence GRGDS, SEQ ID NO: 2), osteogenic growth peptide subunit (OGP[10-14], sequence YGFGG, SEQ ID NO: 3) and/or bone morphogenic protein-2 (BMP-2, sequence KIPKASSVPTELSAISTLYL, SEQ ID NO: 4). As noted above, lysine may be employed at each end of the PEP and may be added to disclosed sequences by methods that would ordinarily occur to one of ordinary skill in the art and be suitably derivitized.

In one embodiment, the peptide-based crosslinker has the following structure:

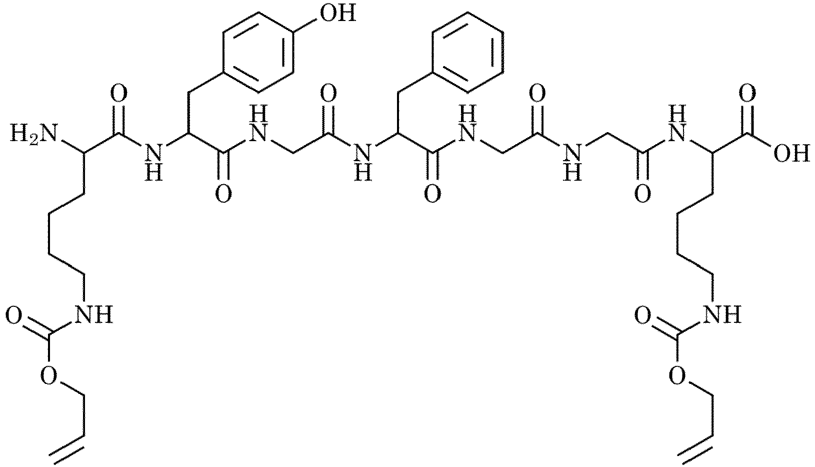

The peptide-crosslinker may be generated by placing lysine amino acid residues at both the N-terminus and C-terminus of the target peptide, for example, as indicated by PEP, during a solid phase synthesis approach. Lysine side chain amino acids may be pre-derivatized with allyloxycarbonyl (Aloc) units during a solid phase synthetic approach. Prior to cleavage from the resin, the N-terminus of the growing peptide may be acetylated. This can yield a functionalized peptide-based crosslinker with two vinyl groups.

The amino acid-based poly(ester urea) may be reacted with the peptide crosslinker to create a peptide crosslinked amino acid-based poly(ester urea). The peptide crosslinker can be incorporated between 0.1 to 5.0 mole percent (mol %) without decreasing the mechanical properties of the base poly(ester urea)polymer. The exact location of the crosslinkers within the composite has not been determined experimentally. Without being limited by theory, it has been observed that during the peptide crosslinking process, radical formation *in situ* may be leading to chain scission in the polymer backbone. While one would generally expect rapid loss of mechanical properties, this has unexpectedly not been observed perhaps due to low mole fraction of crosslinker and high initial molecular mass.

The crosslinked amino acid-based poly(ester urea)s of this disclosure may be utilized to create scaffolds, porous scaffolds, fibers, films, webbing and/or meshes. Suitable processing steps include, but are not limited to, compression molding fabrication, injection molding fabrication, and/or electrospinning and may be used, for example, to fabricate medical devices among other articles.

Electrospinning is used to form fibers and/or nanofibers of poly(ester urea) polymers used in methods of the presently claimed invention. Electrospinning may involve preparing a solution comprising a polymer and a solvent; pumping the polymer solution through a syringe or other reservoir through a needle and/or Taylor cone which may be held at an electrical potential; forming a jet of the solution comprising a polymer; and/or and collecting fibers and/or nanofibers on a collector that is electrically grounded. The collector that is used may have various geometries, and this geometry may affect how the fibers are collected on the collector. For example, some collectors used may collect the fibers in random or pseudo-random orientations, while other collectors may collect the polymer fibers in substantially parallel and/or nonrandom orientation. In some embodiments, a flat collector may be used, while in other embodiments, a cylindrical collector that can be rotated may be used. Variations and/or optimizations of an electrospinning process may also be utilized as would normally occur to one of ordinary skill in the art. In one embodiment of the present disclosure, fluorinated solvents such as, but not limited to, hexafluoroisopropanol may be used as a solvent when electrospinning poly(ester urea) polymers of the present disclosure. Other solvents that may be used for electrospinning such polymers include, as examples, acetone, dimethylformamide (DMF), dichloromethane (DCM), hexafluoro-2-propanol, tetrachloroethane, tetrahydrofuran (THF), dimethyl sulfoxide (DMSO), chloroform (CHCl₃), methanol, phenol and/or combinations thereof. When combinations of solvents are used, such combinations include, but are not limited to, combinations of two or more solvents, such as the combinations of acetone and DMF, DMF and DCM, acetone and DMSO, among other combinations. When combinations of solvents are used, each solvent used in the combination can be used in a quantity or percentage of the total composition in any suitable quantity. The solvents and/or mixtures of solvents that may be utilized in embodiments of the present disclosure may be chosen based upon a variety of criteria. Such criteria include, but are not limited to toxicity, volatility, polarity, conductivity, and/or other criteria or combinations of criteria.

Other variables that may be modified as needed during electrospinning include, but are not limited to, the voltage applied to the syringe needle, the voltage and/or ground applied to the collector plate, the electrospinning may be conducted in either a vertical and/or horizontal plane, the concentration of the polymer solution, the electrical conductivity of the polymer solution, the viscosity of the polymer solution, the surface tension of the polymer solution, the flow rate of polymer, the geometry of the collector, the ambient temperature, the ambient humidity, the air velocity in the chamber, the motion of the collector, the size of the collector, the relative motion of the collector, the temperature of the needle, the geometry of the needle tip, and/or the gauge of the needle. Electrospinning techniques used in certain embodiments may be coaxial electrospinning, emulsion electrospinning, and/or melt electrospinning.

In some embodiments, electrospinning of poly(ester urea) polymers may also comprise electrospinning of one or more additional polymers (e.g., a second polymer). For example, in one embodiment, poly(ester urea) polymers are co-spun with another water-soluble polymer, for example poly(ethylene oxide). When co-spinning is performed, the polymers may be spun from different reservoirs and/or different Taylor Cones, and/or may be spun by coaxial electrospinning. When coaxial electrospinning is utilized, a water-soluble polymer will typically be the outer layer, while a poly(ester urea) polymer may be used in the inner layer, however, the opposite configuration may also be used where the inner layer comprises a water-soluble polymer while the outer layer comprises a poly(ester urea) polymer. After electrospinning, the polymer fibers may be washed with a composition comprising water to dissolve and/or remove a water-soluble polymer, additional and/or larger pore sizes in the polymer fibers may be observed.

In another embodiment, a water-soluble composition may be added to a composition comprising poly(ester urea) polymers. After such a composition is spun into fibers and washed with a composition comprising water, additional and/or larger pore sizes may be observed in the fibers. Water-soluble compositions that may be utilized in embodiments of the present disclosure include, but are not limited to salts such as sodium chloride (NaCl) and/or carbohydrates, including but not limited to sucrose.

When electrospinning is utilized to form polymer fibers of the present disclosure, such fibers may be termed "nanofibers" or simply "fibers." These fibers may have various dimensions, and in the methods presently claimed, the maximum diameter of the fibers is 10 micrometers (µm). In embodiments, the maximum diameter is 8 µm, 5 µm, or 1 µm.

In some embodiments, the fibers have a longitudinal axis, which may be randomly oriented.

In some embodiments, the size of space between polymer fibers, or pores, may be measured. In preferred embodiments, pore size is measured using NIH Image J software (available at http://imagej.nih.gov/ij/index.html) and the Diameter J plugin (available at http://fiji.sc/DiameterJ) for NIH Image J. In some embodiments, the pore side of nanofibers that were electrospun in random orientation and sterilized with poly(ethylene oxide) were measured. For poly(1-PHE-6) poly(ester urea) nanofibers, the maximum pore area has been measured to be about 39 µm² without coaxial spinning. For poly(1-PHE-6)/PEO nanofibers that were coaxially electrospun, the pre area was measured to be about 104 µm². For poly(1-VAL-10) nanofibers that were electrospun, a maximum pore area of about 32 µm² was observed. Poly(1-VAL-10)/PEO nanofibers that were coaxially electrospun had a maximum pore area of about 304 µm². In other embodiments, when the concentration of polymer solution was varied during electrospinning, this had an effect on the observed pore size. When the concentration of poly(1-VAL-10) was varied from about a 10% w/w concentration of electrospinning solution to about 20% w/w concentration of electrospinning, the pore area increased from about 32 µm² to about 118 µm². In some embodiments, the polymer comprising poly(ester urea) polymers, the medical devices disclosed, and/or combinations thereof may have a porosity of between about 20 percent to about 75 percent. In other embodiments, the polymer comprising poly(ester urea) polymers, the medical devices disclosed, and/or combinations thereof may have a porosity of between about 30 percent to about 65 percent. And in still other embodiments, the polymer comprising poly(ester urea) polymers, the medical devices disclosed, and/or combinations thereof may have a porosity of between about 40 percent to about 55 percent.

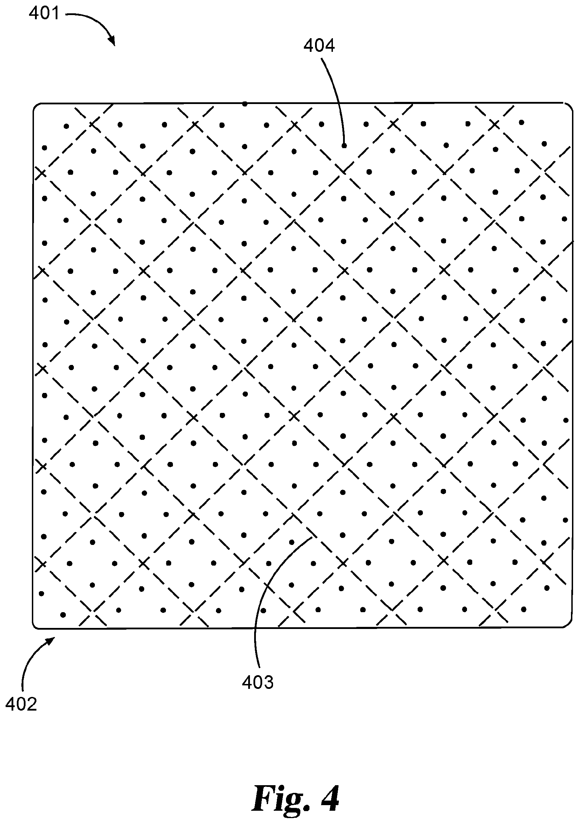

The polymers of the present disclosure may be formed into medical or research related articles, or components thereof. In certain embodiments, a soft tissue support mesh material, such as a hernia repair mesh, may be comprised partially or fully from polymers and/or polymer fibers of the present disclosure. Such grafts may be implanted into a patient to repair, support, and/or reinforce bodily structures. Figure 4 shows a top view of one embodiment of a hernia repair graft material that may be implanted into a patient. FIG. 4 shows a top view of a hernia graft portion (401), and a stitching pattern (403) comprising a series of parallel suture lines (403) spaced about 2.5 cm apart. In this illustrative embodiment, the suture lines (403) are spaced between every other row of apertures (404) that are formed in the graft (401) (typically to allow passage of fluid therethrough) so that each 'diamond' formed by suture lines (403) includes a four-perforation cluster therein. In addition to diamond shapes, in certain embodiments, other stitched shapes will be constructed which fully or partially surround one or more perforations occurring in the material. These shapes include circles, ovals, rectangles and various other shapes having curvilinear and/or rectilinear features.

Polymers and/or polymer fibers of the present disclosure may be incorporated into medical products, such as tissue support meshes, and sterilized. Such tissue support meshes or other products may have an elastic modulus of greater than or equal to about 5 MPa or 15 MPa, and typically in the range of about 20 MPa to about 80 Mpa when a solid cross-sectional area is assumed, and does not account for porosity throughout the thickness of the materials in the measurements. Such tissue support meshes may be provided in sheet and/or any other suitable form.

Medical devices of the present disclosure may be packaged and then terminally sterilized and/or may be sterilized and then packaged. In certain embodiments of the present disclosure, a material may be sterilized so as to not significantly alter the properties of the material. Sterilization may be performed by the step of applying radiation, for example, including but not limited to, ionizing radiation, non-ionizing radiation, gamma rays, or electron beams.

Further, the manufacturing process and/or sterilization process may comprise other steps in addition to sterilizing steps. Such steps may include electrospinning, including for example but not limited to, electrospinning of polymer fibers, melting, molding, packaging (e.g., medically acceptable packaging), and/or washing steps.

According to the presently claimed method, electron beam (e-beam) is used as a source of radiation and directed towards a material, the electron beam having an energy level of 5 MeV to 20 MeV. In embodiments, an electron beam may have an energy level of about 5 MeV to about 15 MeV. In still another embodiment, an electron beam may have an energy level of about 10 MeV. In some embodiments, the electron beam comprises substantially single energy good quality spectral performance. In other embodiments, the electron beam does not have a substantially single energy good quality spectral performance. However, any suitable energy level may be chosen as appropriate, as those skilled in the art will appreciate that the energy of the electron beam may affect the depth of penetration of the radiation, and/or may not affect the exposure time needed, and that the appropriate energy level of the electron beam is dependent at least in part on the dimensions of the specific material to be irradiated.

According to the presently claimed method, the material is irradiated with an electron beam and the material receives a dosage of radiation between 5 kGy and 50 kGy. In embodiments, the material is irradiated with a dosage of about 10 kGy to about 35 kGy. In still another embodiment, a material is irradiated with a dosage of greater than about 25 kGy. This irradiation can be selected to be sufficient to sterilize the material.

Sterilization or sterilized, as used herein, means a process or state of removing, destroying, or otherwise freeing a material of viable microorganisms. According to the presently claimed method, the sterilization process attains a sterility assurance level (SAL) with a probability of less than or equal to about 10⁻⁶ (i.e., referring to a condition wherein the material has a bioburden such that the probability of having one living microorganism (CFU) on and/or in a given section of the material is one in one-million or less).

When poly(ester urea) polymers of the present disclosure are irradiated with an electron beam, the molecular weight of the polymer may be affected. In some embodiments, the molecular weight of the polymer may decrease by about 5 percent to about 20 percent, more typically about 10 percent to about 15 percent. In one embodiment, the molecular weight of poly(1-PHE-6) polymers changed from about 48,000 Daltons to about 42,000 Daltons after sterilization by electron beam sterilization. In another embodiment, the molecular weight of poly(1-VAL-10) polymers changed from about 61,000 Daltons to about 55,000 Daltons after sterilization by electron beam sterilization. Additionally or alternatively, the molecular weight distribution or polydispersion index (PDI), which is measured by dividing the mass average molecular weight by the number average molecular weight, may also be affected. In certain embodiments, the molecular weight distribution may vary by about 0 percent to about 20 percent from before sterilization to after sterilization. For example, in one embodiment the PDI for poly(1-VAL-10) changed from about 1.7 to about 1.5 from before sterilization to after sterilization. In another embodiment, the PDI for poly(1-PHE-6) remained about the same from before sterilization to after sterilization. Additionally or alternatively, materials (*e*.*g*., electrospun fiber materials) incorporating poly(ester urea) polymer fibers as disclosed herein may exhibit a decrease in tensile strength caused by exposure to the radiation. In certain embodiments herein, such decrease in tensile strength is less than about 35 percent, and typically in the range of about 25 percent to about 32 percent. In one embodiment, the average elastic modulus decreased from about 31 MPa to about 23 MPa for poly(1-PHE-6) polymer fibers. In another embodiment, the average elastic modulus decreased from about 65 MPa to about 45 MPa for poly(1-VAL-10) polymer fibers.

Materials, for example sheet materials, incorporating poly(ester urea) fibers as discussed herein, can be used alone in medical or other devices, or can be combined with other materials (*e*.*g*., other sheet materials) in medical or other devices. In certain embodiments, a sheet material incorporating the poly(ester urea) fibers is combined with one or more, or two or more, additional sheet materials to form an overall laminated sheet device. The sheets of the laminated sheet device can be held together by bonding and/or by stitching (*e*.*g*., in a stitching pattern as discussed in conjunction with Figure 4, the other features of which may also be included). Such additional sheet materials can in some variants herein be extracellular matrix sheet materials. In this regard, embodiments contemplated herein include a sheet material incorporating the poly(ester urea) fibers sandwiched between at least a first extracellular matrix sheet material and a second extracellular matrix sheet material in a laminate device. The sheets of the laminate device can be held together by any suitable material or method, including for example by fusing, bonding, adhering, and/or stitching the sheets together. When used, the extracellular matrix sheets can be provided by intact segments of decellularized collagenous tissue membrane, such as membranous collagenous extracellular matrix (ECM) materials. For example, suitable membranous ECM materials include as examples those comprising submucosa, renal capsule membrane, dermal collagen, dura mater, pericardium, fascia lata, serosa, subserous fascia, amnion, peritoneum or basement membrane layers, including liver basement membrane. Suitable submucosa materials for these purposes include, for instance, intestinal submucosa including small intestinal submucosa, stomach submucosa, urinary bladder submucosa, and uterine submucosa. These or other ECM materials can be characterized as membranous tissue layers harvested from a source tissue and decellularized. These membranous tissue layers can have a porous matrix comprised of a network of collagen fibers, wherein the network of collagen fibers preferably retains an inherent network structure from the source tissue. In particular aspects, collagenous matrices comprising submucosa (potentially along with other associated tissues) useful in the present invention can be obtained by harvesting such tissue sources and delaminating the submucosa-containing matrix from smooth muscle layers, mucosal layers, and/or other layers occurring in the tissue source, and decellularizing the matrix before or after such delaminating . For additional information as to some of the materials useful in the present invention, and their isolation and treatment, reference can be made, for example, to U.S. Patent Nos. 4,902,508, 5,554,389, 5,993,844, 6,206,931, and 6,099,567, 8,541,372 and 9,044,455. Submucosa-containing or other ECM tissue, when used in the invention, is also preferably highly purified, for example, as described in U.S. Patent No. 6,206,931 to Cook et al. Thus, preferred ECM material will exhibit an endotoxin level of less than about 12 endotoxin units (EU) per gram, more preferably less than about 5 EU per gram, and most preferably less than about 1 EU per gram. As additional preferences, the submucosa or other ECM material may have a bioburden of less than about 1 colony forming units (CFU) per gram, more preferably less than about 0.5 CFU per gram. Fungus levels are desirably similarly low, for example less than about 1 CFU per gram, more preferably less than about 0.5 CFU per gram. Nucleic acid levels are preferably less than about 5 µg/mg, more preferably less than about 2 µg/mg, and virus levels are preferably less than about 50 plaque forming units (PFU) per gram, more preferably less than about 5 PFU per gram. These and additional properties of submucosa or other ECM tissue taught in U.S. Patent No. 6,206,931 may be characteristic of any ECM tissue used in the present invention. As well, submucosa-containing or other membranous ECM tissue material may retain one or more growth factors native to the source tissue for the tissue material, such as but not limited to basic fibroblast growth factor (FGF-2), transforming growth factor beta (TGF-beta), epidermal growth factor (EGF), cartilage derived growth factor (CDGF), and/or platelet derived growth factor (PDGF). As well, submucosa or other ECM materials when used in the invention may retain other bioactive agents native to the source tissue, such as but not limited to proteins, glycoproteins, proteoglycans, and glycosaminoglycans. For example, ECM materials may include native heparin, native heparin sulfate, native hyaluronic acid, native fibronectin, native cytokines, and the like.

In order to promote a further understanding of the present invention and its various embodiments, the following specific examples are provided. It will be understood that these examples are illustrative and not limiting of the invention.

### Materials.

Unless listed otherwise, all solvents and reagents were purchased from Sigma-Aldrich and used as received. Fluorenylmethyloxycarbonyl (FMOC)-protected amino acids and preloaded Wang resins were purchased from CEM Corp. Alpha minimum essential medium (α-MEM) and ultra culture media were purchased from Lonza. All other cell culture reagents were purchased from Invitrogen Corp. All reagents were used as received.

### EXAMPLE 1

### Synthesis of Di-p-toluenesulfonic acid salts of Bis-L-phenylamine and Bis-L-leucine esters

Di-p-toluene sulfonic acid salts of Bis-L-phenylamine and Bis-L-leucine esters were prepared using procedures as discussed in Pang X., et al., Synthesis, Characterization and Biodegradation of Functionalized Amino Acid-based Bioanalogous Polymers., Biomaterials 2010, 31, 3745. Briefly, L-Leucine (1.31 g, 10 millimoles (mmol)), 1,6-hexanediol (0.48 grams (g), 4 mmol), p-toluenesulfonic acid (1.92 g, 10 mmol), and toluene (20 millileters (mL)) were mixed in a 250 mL 3-neck flask equipped with a Dean Stark trap and a magnetic stir bar. The system was purged with nitrogen for 30 minutes (min) after which the reaction mixture was heated at 135 °C under nitrogen for 20 hours (h). The reaction mixture was allowed to cool to ambient temperature and the crude product was isolated by vacuum filtration. The organic residue was recrystallized four times using 25 mL water to yield 2.26 g (82 %) of di-p-toluenesulfonic acid salt of Bis-L-leucine ester as a white power. The product was characterized with ¹H-NMR, ¹³C-NMR, and melting point measurements.

*Di-p-toluenesulfonic acid salt of Bis-L-leucine hexane-1,6-diester* (Monomer 1, l-LEU-6): mp: 186-188 °C; ¹H-NMR (300 MHz, DMSO-d₆): δ = 0.90 (d, 12H), 1.34 (s, 4H), 1.45-1.80 (m, 8H), 2.29 (s, 6H), 3.99 (t, 2H), 4.15 (d, 4H), 7.13 (d, 4H), 7.49 (d, 4H), 8.31 (s, active H); ¹³C-NMR (75 MHz, DMSO-d₆): δ = 169.91, 145.34, 137.35, 129.10, 125.48, 65.52, 50.62, 27.76, 24.75, 23.79, 23.13, 21.92, 20.79.

*Di-p-toluenesulfonic acid salt of Bis-L-phenylalanine hexane-1,6-diester* (Monomer 2, l-PHE-6): mp: 215-217 °C; ¹H-NMR (300 MHz, DMSO-d₆): δ = 0.90-1.15 (m, 4H), 1.38 (s, 4H), 1.25-1.50 (m, 4H), 2.23 (s, 6H), 2.91-3.09 (m, 2H), 3.10-3.21 (m, 2H), 4.01 (t, 4H), 4.30 (t, 2H), 7.1 1 (d, 4 H), 7.19-7.40 (m, 10H), 7.49 (d, 4H), 8.43 (s, active H); ¹³C-NMR (75 MHz, DMSO-d₆): δ = 169.06, 145.00, 138.12, 134.70, 129.32, 128.55, 128.21, 127.54, 125.53, 65.45, 53.34, 36.20, 27.63, 24.70, 20.82.

### EXAMPLE 2

### Interfacial Polycondensation of l-LEU-6 and l-PHE-6

A general scheme for poly(ester urea) synthesis is described in Figure 1. Monomer l-LEU-6 (6.89 g, l0 mmol), sodium carbonate (3.18 g, 30 mmol), and water (150 mL) were mixed in a 500 mL 3-neck flask equipped with an overhead mechanical stirrer and a thermometer. The mixture was heated with a warm water bath at 40 °C for 30 min. The water bath was removed and replaced with an ice-salt bath. When the inside temperature decreased to about 0 °C, pre-prepared triphosgene solution (1.035 g, 3.30 mmol in 30 mL chloroform) was added to the reaction system quickly (5 seconds) with fast mechanical stirring. The reaction was allowed to proceed for 30 min and then additional aliquots of triphosgene (0.108 g, 0.330 mmol, total) were dissolved in chloroform (5 mL) and 1 mL aliquots were added into the reaction system every 10 min. After the addition of triphosgene, the organic phase was precipitated into hot water, filtered, and dried in vacuum to yield a white solid (3.2 g, 74.5 % yield). The product was characterized by ¹H-NMR, ¹³C-NMR, FT-IR spectroscopy, size exclusion chromatography (SEC), thermogravametric analysis (TGA), and differential scanning calorimetry (DSC). Molecular weight and thermal properties of polymers are listed in Table 1.

*Poly (l-LEU-6):* FT-IR (cm⁻¹): 1740 [-C(CO)-0-], 1648, 1542 [-NH-C(O)-NH-], 3283 [-NH-C(0)-NH-]; ¹H-NMR (300 MHz, DMSO-d₆): δ = 0.91 (d, 12H), 1.20-2.00 (m, 14H), 4.21 (t, 4H), 4.45 (d, 2H), 5.35-5.80 (m, active H); ¹³C-NMR (75 MHz, DMSO-d₆): δ 74.64, 157.08, 65.00, 51.60, 65.00, 51.00, 42.31, 28.20, 25.29, 24.74, 22.61, 22.12.

*Poly (l-PHE-6):* FT-IR (cm⁻¹): 1736 [-C(CO)-0-], 1649, 1553 [-NH-C(O)-NH-], 3384 [-NH-C(0)-NH-]; ¹H-NMR (300 MHz, DMSO-d₆): δ =; 1.15 (m, 4H) 1.43 (m, 4H) 2.5(DMSO) 2.87-2.94 (m, 4H) 3.94 (m, 4H) 4.34-4.40 (m, 2H) 6.47-6.5 (m, 2 H) 7.14-7.17 (m, 4H) 7.19-7.28 (d, 6H); ¹³C-NMR (75 MHz, DMSO-d₆): δ = 25.33, 28.38, 38.10, 39.40-40.56 (DMSO), 54.49, 64.65, 127.04, 128.78, 129.65, 137.34, 157.07, 172.89.

### EXAMPLE 3

### Alternative Polymerization of l-LEU-6 and l-PHE-6

A synthesis of homo-poly(ester urea)s, without using diisocyanates, via active polycondensation is discussed by Katsarava et al., Amino Acid-based Bioanalogous polymers., Synthesis and Study of Regular Poly(ester amide)s based on Bis(alpha-amino acid) Alpha, Omega-alkyne Diesters, and Aliphatic Dicarboxylic Acids., J. Polym. Sci. Part A: Polym Chem 1998, 37, 391. In this procedure, active carbonates (*e*.*g*., di-p-nitrophenyl carbonate) interact with di-p-toluenesulfonic acid salts of bis(α-amino acid)-α,ω-alkylene diesters. The molecular weight can be tailored by altering the molecular weights of the constituent monomers and the degree of polymerization. This experiment used a modified version of the process for the synthesis of the base poly(ester urea) materials which are described as x-amino acid-y where x and y are the number of carbon atoms in the chain as shown in Figure 1. The leucine (LEU) and phenylalanine (PHE) amino acid-based poly(ester urea)s utilized 1,6-hexane diol, and are denoted l-LEU-6 and l-PHE-6. The molecular weight, molecular weight distribution and thermal properties of the poly(l-LEU-6) and poly(1-PHE-6) were measured.

At ambient temperature, poly(1-PHE-6) is not soluble in conventional organic solvents, but is soluble in hexafluoroisopropanol and 3:1 mixtures of tetrachloroethane:phenol. When the poly(l-LEU-6) was melt processed, the glass transition temperature (T_{g}) remained the same but no melting peak is observed indicating that no crystallinity was present. This indicates that polymer crystallinity can be suppressed using the appropriate processing method. The degradation temperatures (T_{d}) of the poly( l-LEU-6) and poly( l-PHE-6) materials were over 100 °C higher than the melting temperature indicating that both materials can be melt processed with limited impact on thermal degradation. These characteristics afford processing techniques such as molding, melt processing, and/or electrospinning to be used to fabricate scaffolds or other medical devices.

### EXAMPLE 4

### Polymer Characterization

### Molecular Weight.

Number-average (Mₙ) and weight-average (M_{w}) molecular weights and molecular weight distribution (M_{w}/Mₙ) were determined by size exclusion chromatography (SEC). The instrument was equipped with a guard column and set of 50 angstroms (Å), 100 Å, 104 Å, and linear (50-104 Å) Styragel 5 µm columns, a Waters 486 tunable UV/vis detector, and a Waters 410 differential refractometer. All the analyses were carried out with a flow rate of 1 mL/min using a RI detector. DMF was used as the eluent at 50 °C. The respective molecular weights and molecular weight distributions of each polymer were determined using a universal calibration curve, which was obtained by plotting ln-[η]/Mₙ (which is the natural log of the intrinsic viscosity divided by the number average molecular weight) as a function of elution volume, after calibration with polystyrene standards (Polymer Laboratories).

### Thermal Characterization.

The degradation temperatures (T_{d}) of poly(l-LEU-6) and poly(1-PHE-6) materials were determined by thermogravimetric analysis (TA instruments, Q50 TGA) across a temperature range of 30 °C to 500 °C at a scanning rate of 20 °C/min under nitrogen. The thermal transitions of the poly(l-LEU-6) and poly(l-PHE-6) materials were characterized by differential scanning calorimetry (TA Instruments, Q2000 DSC) at a scanning rate of 10 °C/min.

### Crosslinked Scaffold Fabrication.

Polymer plugs were prepared by a compression molding fabrication process. Polymer materials about l g, and a corresponding amount of peptide crosslinker and photoinitiator Irgacure 2959 were dissolved in 20 mL hexafluoride-2-propanol. The clear solution was photo-irradiated with 365 nm UV light for 45 minutes. The solvent was evaporated in a fume hood at room temperature for 24 h followed by vacuum drying at 80 °C for 24 h. The composite was crushed into small pieces and melt-pressed into sheets using a Carver Hydraulic unit model 3912 with a pressure of 12,41 MPa (1800 psi) at the designed temperature (130 °C for poly(l-LEU-6) and 180 °C for poly(l-PHE-6)). The polymeric block was cooled to room temperature, and annealed under vacuum at the appropriate temperature for 24 h (80 °C for poly(l-LEU-6) and 130 °C for poly(l-PHE-6)). The final polymeric block was cut into small circular plugs with diameters measuring 0.5 centimeters (cm) for testing. All plugs were stored in nitrogen atmosphere at -20 °C for future use.

### Mechanical Characterization.

Dynamic Mechanical Analysis (DMA): The Young's moduli of the poly(l-PHE-6), 0.5% OGP poly(l-PHE-6), and 1.0% OGP poly(1-PHE-6) data were determined using a TA Q800 dynamic mechanical analysis (DMA) instrument with sample dimensions 40x2.0x0.2 mm at ambient temperature. The strain rate was 1.5% sec⁻¹. Using small strains (<0.15%) the Young's moduli were determined using the slope of the tangent line in the linear regime. Stress strain data were reported using the TA Universal Analysis software. The data were plotted in Origin 8 and Young's modulus values were calculated using regression analysis in the linear regime. Values for Young's moduli and standard deviations were determined from four individual measurements.

Instron: The elastic modulus and tensile properties of the poly(l-LEU-6) and the poly(1-PHE-6) were measured using an Instron 3365 universal materials testing machine. The gauge length was 20 millimeters (mm) and the crosshead speed was set at 30 mm/min.

These data can be summarized as follows in Table 1 and Table 2.

**TABLE 1: Characterization data summary for amino acid-based poly(ester urea).**

| **Samples** | **M_{W}** | **M_{w}/Mₙ** | **T_{g} (°C)** | **Tₘ** (°C) | **T_{d} (°C)** | **G' (gigapascals (GPa))** |
|---|---|---|---|---|---|---|
| Poly(1-LEU-6) | 76,800 | 2.12 | 57 | 126 | 275 | 4.4 ± 0.9 |
| Poly(1-PHE-6) | 84,000 | 2.42 | 107 | 153 | 335 | 6.1 ± 1.1 |

**TABLE 2: Summary of mechanical properties of peptide-crosslinked poly(ester urea) polymers**

| | **Instron Testing** | | | **Dynamic Mechanical Analysis Young's Modulus** | | | | |
|---|---|---|---|---|---|---|---|---|
| **Samples** | **Elastic Modulus (GPa)** | **Tensile Strain (%)** | **Tensile Stress** | **Trial 1** | **Trial 2** | **Trial 3** | **Trial 4** | **Average (± Std. Dev.)** |
| poly(l-LEU-6) | 4.4 ± 0.9 | 470 ± 50 | 43 ± 9 | | | | | |
| poly(1-PHE-6) | 6.1 ± 1.1 | 510 ± 30 | 45 ± 3 | 3.38 | 3.04 | 2.94 | 2.82 | 3.05 ± 0.24 |
| poly(1-PHE-6) 0.5% OGP-based crosslinker | | | | 3.50 | 3.39 | 3.37 | 3.40 | 3.41 ± 0.06 |
| poly(1-PHE-6) 1.0% OGP-based crosslinker | | | | 4.30 | 4.27 | 4.00 | 4.10 | 4.18 ± 0.14 |

### EXAMPLE 5

### Electrospinning

Solutions for electrospinning were prepared one day prior to spinning by making a 10%-20% by weight (w/w) solution of poly(ester urea) polymer in 1,1,1,3,3,3-hexafluoroisopropanol (HFIP). Electrospinning was carried out using a teflon reservoir with a 25-20 gauge needle depending on the solution viscosity. Positive voltage was applied to the reservoir at about 10 kV. The collector was grounded and covered in a sheet of foil. A polymer fiber sheet was collected on the collector and water was used to detach the polymer fiber sheet from the collector. The polymer fiber sheet was freeze-dried prior to use. Scanning electron microscopy was used to quantify the polymer fiber diameter and to examine the fiber morphology.

### EXAMPLE 6

### Histology and Histomorphmetric Analysis

Tissue sections (5 µm) were cut (Leica RM2235 micrometer) and stained by hematoxylin and eosin (Ventana ST5020 Automated Stainer, Hematoxylin 7211 and Eosin 71204) to show normal tissue architecture, Mallory's trichrome (Ventana NEXES Special Stains, Trichrome II Staining Kit 860-013) for collagen deposition and cellular infiltration, and Alizarin red to detect mineralization of calcium as evidence of bone cell activity. For Alizarin Red S staining, histological sections stained with 40 millimolar (mM) Alizarin Red S solution, pH 4.2 at ambient temperature for 10 min, rinsed five times in distilled water and washed for 15 min in PBS. Histological sections were counter-stained with hematoxylin, dehydrated with ethanol and rinsed in xylene before mounting with permamount. All slides were examined with an Olympus BX51 light microscope to identify the location of the polymer within the subcutaneous region and digital images were captured using the Qlmaging camera and software. Histomorphometric features were analyzed on the digital images of the Trichrome sections at 40X using BioQuant Nova Prime Image Analysis software (v.6.75.10; Nashville, TN). The total area in square micrometers (µm²) of connective tissue surrounding and including the polymer was delineated as the region of interest. A separate area measurement (µm²) was defined for the regions of tissue extending from the connective tissue capsule into the region containing the polymer, indicating polymer degradation. Within the region of interest, the threshold was selected to identify the regions of red stain and a video count array measured the total pixel area (µm²) of collagen deposition/cellular infiltration. The percentages of degradation and cellular infiltration were calculated in relation to the respective total area region of interest. The width of the connective tissue capsule was measured at 5 random locations surrounding the polymer and averaged. The numbers of giant cells and blood vessels were counted within and adjacent to the region of interest.

### EXAMPLE 7

### Sterilization of poly(ester urea) polymers by ethylene oxide (not presently claimed) and electron beam (presently claimed)

Samples of poly(1-PHE-6) and poly(1-VAL-10) were sterilized by ethylene oxide treatment and electron beam treatment. A Anprolene AN74i tabletop sterilizer was utilized in certain procedures. Ethylene oxide sterilization was also used for sterilization of ECM materials in a low temperature sterilization chamber for 200 minutes. E-beam irridation of poly(ester urea) nanofibers was carried out with a dose of 35 kGy.

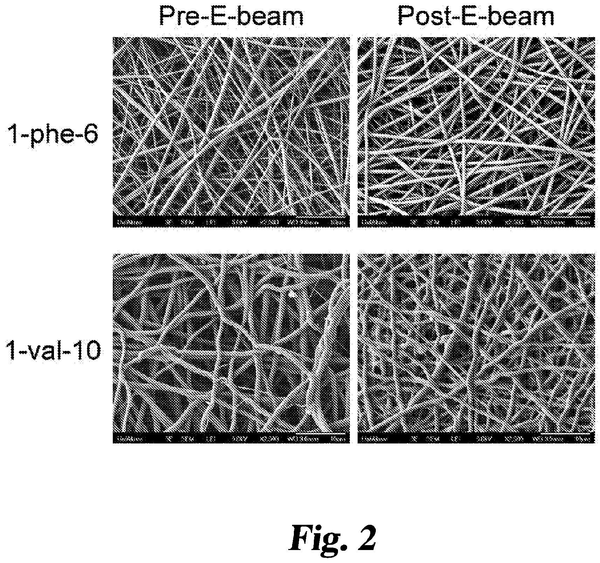

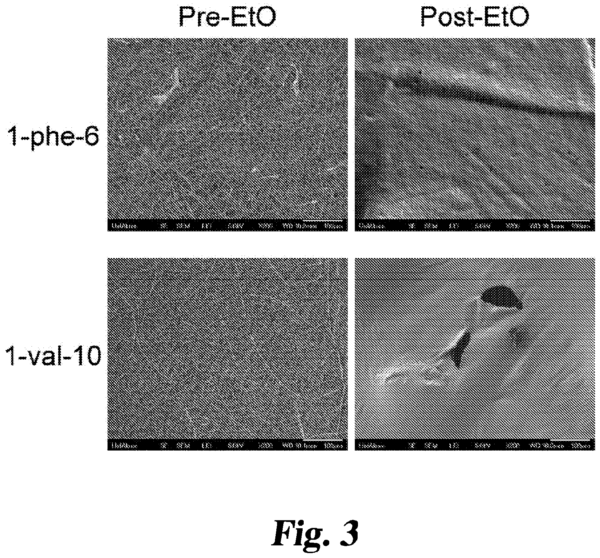

Figure 2 and Figure 3 show digital images of scanning electron microscope images of fibers formed by electrospinning before and after sterilization by treatment with ethylene oxide and electron beam. As can be seen by these figures, when ethylene oxide is used to sterilize the polymer fibers, the morphology and/or structure of the fibers are damaged. However, when sterilization by electron beam is performed, the polymer fibers remain intact, meaning that fibers are still identifiable and/or that the average diameter of the polymer fibers change by no more than about 20 percent when viewed in an electron micrograph. Table 3 and Table 4 summarize the molecular weight and molecular weight distribution of these polymers pre-sterilization and post-sterilization by electron beam.

**TABLE 3. Molecular weights and molecular weight distribution of poly(1-PHE-6) pre-sterilization by electron beam and post-sterilization by electron beam.**

| | poly(1-PHE-6) pre-sterilization | poly(1-PHE-6) post-sterilization |
|---|---|---|
| Mₙ | 31,087 | 26,208 |
| M_{W} | 48,452 | 42,145 |
| Mₙ/M_{W} | 1.559 | 1.608 |

**TABLE 4. Molecular weights and molecular weight distribution of poly(1-VAL-10) pre-sterilization by electron beam and post-sterilization by electron beam.**

| | poly(1-VAL-10) pre-sterilization | poly(1-VAL-10) post-sterilization |
|---|---|---|
| Mₙ | 35,694 | 35,737 |
| M_{W} | 61,092 | 54,759 |
| Mₙ/M_{W} | 1.712 | 1.532 |

**TABLE 5. Tensile Modulus of poly(ester urea) polymer fibers and films pre-sterilization by electron beam and post-sterilization by electron beam.**

| 1-PHE-6 Fibers | | |
|---|---|---|
| | Modulus (megapascals (MPa)) | Toughness |
| Pre-sterilization | 31.20 ± 8.83 | 3.98 ± 1.84 |
| Post-sterilization | 22.96 ± 4.74 | 3.68 ± 3.23 |

| 1-VAL-10 Fibers | | |
|---|---|---|
| | Modulus (MPa) | Toughness |
| Pre-sterilization | 64.52 ± 7.67 | 25.72 ± 17.95 |
| Post-sterilization | 45.17 ± 12.04 | 9.85 ± 7.81 |

| 1-VAL-10 Film | | |
|---|---|---|
| | Modulus (MPa) | |
| | 300.81 ± 164.59 | |

### EXAMPLE 8

### Cell Proliferation

Poly(ester urea) polymer fiber sheets were cut into 1 centimeter (cm) x 1 cm squares or 0.9 cm diameter circles and extracellular matrix (ECM) samples were cut into 1 cm diameter circles. ECM samples were sterilized by EtO sterilization while the poly(ester urea) polymer fiber samples were sterilized by e-beam sterilization. After sterilization, samples were soaked in medium comprising Dulbecco's modified eagles medium (DMEM) with 1% penicillin/streptomycin and 10% fetal bovine serum (FBS) and 1 millimolar (mM) sodium pyruvate for three nights in cell culture conditions (37 °C, 5% CO₂, 95% humidity) to allow serum proteins to attach to the samples.

NIH3T3 fibroblasts were seeded onto the samples. For seeding, cells were trypsinized with 0.05% trypsin ethylenediaminetetraacetic acid (EDTA), collected, centrifuged at 3,000 rpm for one minute to obtain a cell pellet, resuspended in medium, and counted using a hemacytometer with trypan blue exclusion. A seed density of 15,000 cells per square centimeter (cells/cm²) was used for seeding cells to the entire area of a 24-well plate and was suspended in 1mL/well. The entire 1 mL aliquot of cells was plated into each well, and the entire plate was agitated to ensure even distribution of cells over the entire sample and well bottom. Plates were incubated in standard cell culture conditions, and were fed fresh medium every other day.

Cell viability was carried out using a live/dead assay after 24 h of incubation. Cell spreading was performed using cytoskeletal and nuclear fluorescent labeling after 3 days of culture. Cell proliferation was assayed using a PrestoBlue metabolic assay on days 3, 7, and 14 with cell number quantification using a standard curve of known cell concentrations seeded into tissue culture wells one day prior to the assay. Control samples without cells were prepared in order to observe absorbance of the scaffolds alone after incubation in the PrestoBlue reagent. Blank wells were also given serum-containing media with the standard curve to obtain background absorbance values of the well. Sample size was n=3 for all samples at each time point for each assay, with n=2 for ECM controls and for cell-free proliferation control samples. Two standard curves were used from each timepoint for proliferation.

For cell viability analysis, the media was aspirated from sample wells and was replaced with a live/dead solution comprising calcein AM to stain live cells green and ethidium homodimer to stain dead cells red. After ten minutes of incubation, samples were imaged using a fluorescent microscope under the TRITC and FITC channels to obtain 20-30 images of each specimen. The images were overlayed and live and dead cells were counted. The number of live cells was divided by the number of total cells to obtain a percentage of cell viability.

For cell spreading analysis, samples were fixed after three days in a culture comprising 3.7% paraformaldehyde in cytoskeleton stabilization (CS) buffer for 10 minutes at 37 °C, and then permeabilized in 0.5% Triton-X-100 for 10 minutes at 37 °C. Samples were rinsed three times for five minutes each time in CS buffer at room temperature prior to quenching aldehyde autofluorescence with a freshly prepared solution of 0.05% sodium borohydride in CS buffer for 10 minutes at room temperature. After quenching, non-specific staining was blocked with 5% donkey serum in PBS for 20 minutes at 37 °C. For focal adhesion staining, primary mouse monoclonal anti-vinculin antibody was mixed 1:200 in 1% donkey serum in PBS and was added to wells until samples were submerged overnight for staining at 4 °C. The next day, samples were rinsed three times for five minutes each time in 1% donkey serum in PBS before adding secondary goat anti-mouse IgG 488 antibody (1:400), and rhodamine phalloidin (1:40) for 1 hour at 37 °C in the dark. The stain was then replaced with a DAPI nuclear stain (2 mM) for 20 minutes at 37 °C in the dark, followed by a quick rinse four times in PBS at room temperature. Samples were imaged using the DAPI, FITC, and TRITC channels at 40x magnification. Images were analyzed for cell aspect ratio by dividing the longest stretch of the cell by the shortest width across the cell nucleus. The average aspect ratio per specimen was calculated and specimen averages were calculated to obtain the sample aspect ratio for each sample.

For cell proliferation analysis, a PrestoBlue metabolic assay was used after 3, 7, and 14 days of culture. To obtain a standard curve for calculating cell number, one day prior to the assay, cells of known density were placed into 24 well plates using a serial dilution. The standard curves ranged from 1,000 to 64,000 or 128,000 cells with points on each curve, with eight points on each curve). To seed the curve, cells were collected using 0.05% Trypsin-EDTA, resuspended in medium, counted using a hemacytometer with trypan blue exclusion, and the amount required for each curve was isolated. For example, for seeding 128,000 cells in the first well with 8 curve points, 256,000 cells were suspended in 1 ml of medium, and diluted serially to obtain 128,000 cells in the first well, 64,000 in the second, 32,000 in the third and so on. The plates were incubated with sample plates overnight to allow the cells to attach and their metabolic activity to equilibrate without allowing for cell doubling. The next day, media was removed from all wells, samples were moved to new wells to allow for counting only the cells attached to the samples, and sample controls without cells were also moved to new wells. A 1:10 dilution of presto blue solution was well-mixed and aliquots were added 1 ml per well, and incubated in the dark using standard cell culture conditions for 6 hours. The plate was then aliquotted in triplicate with 100 microliter (µL) per well in a 96 well plate that was then read for fluorescence at 535/615nm excitation/emission with 60% sensitivity. The standard curve was plotted and a linear trendline for calculating cell number from fluorescence readings was observed. Readings for the blank wells were averaged and subtracted from every well. Sample controls without cells were averaged and subtracted from every respective sample well. Triplicates were averaged to obtain a specimen reading, and the specimen averages were averaged for fluorescence of each sample and the standard deviation for each sample was calculated. These numbers were converted to cell number using the standard curve equation.

For cell migration, samples were fixed after 7 and 14 days of culture in 4% paraformaldehyde in PBS for 10 minutes at 37 °C. Samples were then embedded on edge in optimal cutting temperature tissue freezing medium (TFM) at -20 °C in a cryostat. Cross-sections measuring 10 micrometers (µm) in thickness were transferred to slides and stained, mounted and coverslipped using Vectashield Hard-Set Mounting Medium with DAPI. Images of the cross-sections were obtained using DAPI and brightfield channels at 10x magnification with automated montage stitching using Cell Sense software to obtain images of the entire cross-section.

Additionally, poly(ester urea) polymer fiber sheets were sterilized and implanted subcutaneously (such an implantation method not forming part of the claimed subject-matter) in the backs of mice for three weeks to examine tissue infiltration and microangiography. It was noted at the time of sample harvest that no gross inflammatory response was evident. Microangiography images suggest that some angiogenesis occurred in the nanofibers.

The uses of the terms "a" and "an" and "the" and similar references in the context of describing the invention (especially in the context of the following claims) are to be construed to cover both the singular and the plural unless otherwise indicated herein or clearly contradicted by context. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

## Claims

1. A method for sterilizing a material comprising poly(ester urea) polymer fibers, comprising the steps of:
exposing a material comprising poly(ester urea) polymer fibers to radiation to sterilize the material;
wherein the material is an electrospun fiber material incorporating the poly(ester urea) polymer fibers and the maximum diameter of the fibres is 10 µm;
**characterized in that** said radiation is electron beam radiation at a dosage of between 5 kGy and 50 kGy and the electron beam has an energy level of 5 MeV to 20 MeV; and
the method produces a material comprising poly(ester urea) polymer fibers; and the material has a sterility assurance level with a probability level of less than about 10⁻⁶ after the step of exposing the material to radiation.

2. The method of claim 1,
wherein the electron beam radiation has an energy of about 10 MeV; and/or
wherein the step of exposing the material comprising poly(ester urea) polymer fibers to radiation is performed for greater than about 1 min.

3. The method of any preceding claim, wherein the poly(ester urea) polymer has a number-average molecular weight of greater than about 5,000 daltons as determined by size exclusion chromatography after said exposing a material comprising poly(ester urea) polymer fibers to radiation.

4. The method of any preceding claim, wherein the poly(ester urea) polymer has a weight-average molecular weight of greater than about 5,000 daltons as determined by size exclusion chromatography after said exposing a material comprises poly(ester urea) polymer fibers to irradiation by an electron beam.

5. The method of any preceding claim, wherein the poly(ester urea) polymer has the chemical structure: 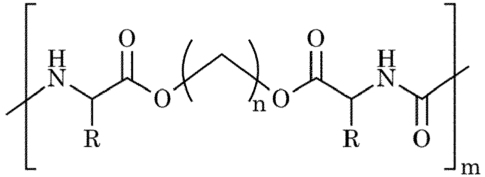 and wherein n and m are independently selected positive integers greater than or equal to 1.

6. The method of claim **5,** wherein n is a positive integer between 1 and 20; optionally wherein n is 6 or n is 10.

7. The method of claim **5,** wherein m is a positive integer between 1 and 1000.

8. The method of any one of claims **5-7,** wherein R is selected from any of the naturally occurring amino acid side-chains; optionally wherein R is selected from the group consisting of isobutyl and benzyl substituents.

9. The method of any preceding claim, wherein said exposing does not reduce the average molecular weight of the poly(ester urea) polymer by more than 50 percent.

10. The method of any preceding claim, wherein said exposing does not reduce the molecular weight distribution by more than 50 percent wherein the molecular weight distribution is calculated by dividing the weight-average molecular weight by the number-average molecular weight.

11. The method of any preceding claim, wherein the difference between the molecular weight distribution of the poly(ester urea) polymer prior to said exposing and after said exposing is less than about 50 percent, wherein the molecular weight distribution is calculated by dividing the weight-average molecular weight by the number-average molecular weight.

12. The method of any preceding claim, wherein the poly(ester urea) polymer is crosslinked prior to said exposing; optionally wherein the crosslinking prior to said exposing is performed using a peptide based crosslinker; and optionally further wherein said peptide based crosslinker has the chemical structure 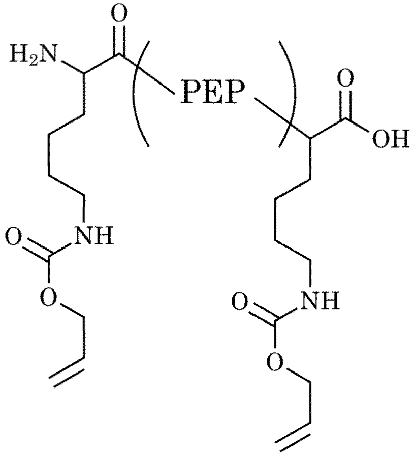 and wherein PEP is a polypeptide chain comprising from 2 to 20 amino acid residues.

13. The method of claim **12,** wherein PEP is a polypeptide selected from the group consisting of SEQ ID NO: 1, SEQ ID NO: 2, SEQ ID NO: 3, and SEQ ID NO: 4.

14. The method of any preceding claim wherein the material after said exposing has an elastic modulus of at least about 5 MPa .

15. The method of any preceding claim wherein said poly(ester urea) polymer fibers further comprise a second polymer that is not a poly(ester urea) polymer; and/or
wherein said poly(ester urea) polymer fibers further comprise a water-soluble composition that is not a poly(ester urea) polymer; and optionally further wherein said water-soluble composition that is not a poly(ester urea) polymer is sodium chloride or sucrose.

16. The method of any preceding claim, wherein said material has a porosity in the range of about 20 percent to about 75 percent.

## Patentansprüche

1. Verfahren zum Sterilisieren eines Materials umfassend Poly(esterharnstoff)polymerfasern, umfassend die Schritte des:
Aussetzens eines Materials, das Poly(esterharnstoff)polymerfasern umfasst, an Strahlung zum Sterilisieren des Materials;
wobei das Material ein elektrogesponnenes Fasermaterial ist, in das die Poly(esterharnstoff)polymerfasern integriert sind, und der maximale Durchmesser der Fasern 10 µm beträgt;
**dadurch gekennzeichnet, dass** die Strahlung Elektronenstrahlstrahlung in einer Dosis zwischen 5 kGy und 50 kGy ist und der Elektronenstrahl ein Energieniveau von 5 MeV bis 20 MeV aufweist; und
durch das Verfahren ein Material hergestellt wird, das Poly(esterharnstoff)polymerfasern umfasst; und das Material ein Sterilitätssicherheitsniveau mit einem Wahrscheinlichkeitsniveau von weniger als etwa 10⁻⁶ nach dem Schritt des Aussetzens des Materials an Strahlung aufweist.

2. Verfahren nach Anspruch 1,
wobei die Elektronenstrahlstrahlung eine Energie von etwa 10 MeV aufweist; und/oder
wobei der Schritt des Aussetzens des Materials, das Poly(esterharnstoff)polymerfasern umfasst, an Strahlung länger als etwa 1 min ausgeführt wird.

3. Verfahren nach einem vorhergehenden Anspruch, wobei das Poly(esterharnstoff)polymer ein zahlendurchschnittliches Molekulargewicht von mehr als etwa 5.000 Dalton, wie durch Größenausschlusschromatografie nach Aussetzen eines Materials, das Poly(esterharnstoff)polymerfasern umfasst, an Strahlung bestimmt, aufweist.

4. Verfahren nach einem vorhergehenden Anspruch, wobei das Poly(esterharnstoff)polymer ein gewichtsdurchschnittliches Molekulargewicht von mehr als etwa 5.000 Dalton, wie durch Größenausschlusschromatografie nach Aussetzen eines Materials, das Poly(esterharnstoff)polymerfasern umfasst, an Strahlung durch einen Elektronenstrahl bestimmt, aufweist.

5. Verfahren nach einem vorhergehenden Anspruch, wobei das Poly(esterharnstoff)polymer die chemische Struktur aufweist 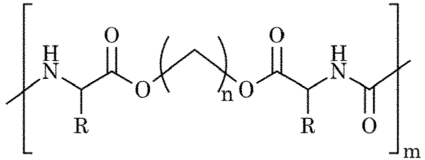 und wobei n und m unabhängig ausgewählte positive ganze Zahlen von mehr als oder gleich 1 sind.

6. Verfahren nach Anspruch 5, wobei n eine positive ganze Zahl zwischen 1 und 20 ist, wobei wahlweise n 6 beträgt oder n 10 beträgt.

7. Verfahren nach Anspruch 5, wobei m eine positive ganze Zahl zwischen 1 und 1000 ist.

8. Verfahren nach einem der Ansprüche 5 bis 7, wobei R unter irgendwelchen der natürlich vorkommenden Aminosäureseitenketten ausgewählt wird, wobei R aus der Gruppe ausgewählt wird bestehend aus Isobutyl- und Benzylsubstituenten.

9. Verfahren nach einem vorhergehenden Anspruch, wobei das Aussetzen das durchschnittliche Molekulargewicht des Poly(esterharnstoff)polymers nicht um mehr als 50 Prozent reduziert.

10. Verfahren nach einem vorhergehenden Anspruch, wobei das Aussetzen die Molekulargewichtsverteilung nicht um mehr als 50 Prozent reduziert, wobei die Molekulargewichtsverteilung durch Teilen des gewichtsdurchschnittlichen Molekulargewichts durch das zahlendurchschnittliche Molekulargewicht berechnet wird.

11. Verfahren nach einem vorhergehenden Anspruch, wobei der Unterschied zwischen der Molekulargewichtsverteilung des Poly(esterharnstoff)polymers vor dem Aussetzen und nach dem Aussetzen weniger als etwa 50 Prozent beträgt, wobei die Molekulargewichtsverteilung durch Teilen des gewichtsdurchschnittlichen Molekulargewichts durch das zahlendurchschnittliche Molekulargewicht berechnet wird.

12. Verfahren nach einem vorhergehenden Anspruch, wobei das Poly(esterharnstoff)polymer vor dem Aussetzen vernetzt wird, wobei wahlweise das Vernetzen vor dem Aussetzen unter Anwendung eines Vernetzers auf Peptidbasis ausgeführt wird und wobei ferner wahlweise der Vernetzer auf Peptidbasis die chemische Struktur aufweist 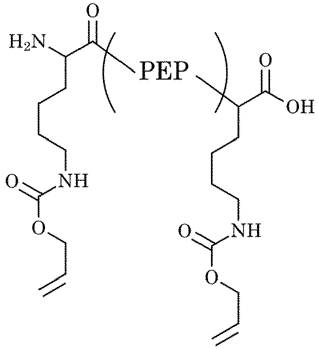 und wobei PEP eine Polypeptidkette ist, die 2 bis 20 Aminosäurereste umfasst.

13. Verfahren nach Anspruch 12, wobei PEP ein Polypeptid ist ausgewählt aus der Gruppe bestehend aus SEQ ID NO: 1, SEQ ID NO: 2, SEQ ID NO: 3 und SEQ ID NO: 4.

14. Verfahren nach einem vorhergehenden Anspruch, wobei das Material nach dem Aussetzen einen Elastizitätsmodul von mindestens etwa 5 MPa aufweist.

15. Verfahren nach einem vorhergehenden Anspruch, wobei die Poly(esterharnstoff)polymerfasern ferner ein zweites Polymer umfassen, das kein Poly(esterharnstoff)polymer ist; und/oder
wobei die Poly(esterharnstoff)polymerfasern ferner eine wasserlösliche Zusammensetzung umfassen, die kein Poly(esterharnstoff)polymer ist; und wobei ferner wahlweise die wasserlösliche Zusammensetzung, die kein Poly(esterharnstoff)polymer ist, Natriumchlorid oder Sucrose ist.

16. Verfahren nach einem vorhergehenden Anspruch, wobei das Material eine Porosität im Bereich von etwa 20 Prozent bis etwa 75 Prozent aufweist.

## Revendications

1. Procédé de stérilisation d'un matériau comprenant des fibres polymères de poly(ester urée), comprenant les étapes consistant à :
exposer un matériau comprenant des fibres polymères de poly(ester urée) à une irradiation pour stériliser le matériau ;
dans lequel le matériau est un matériau de fibres électrofilées incorporant les fibres polymères de poly(ester urée) et le diamètre maximal des fibres est de 10 µm ;
**caractérisé en ce que** ladite irradiation est une irradiation par faisceau d'électrons à une dose comprise entre 5 kGy et 50 kGy et le faisceau d'électrons présente un niveau d'énergie de 5 MeV à 20 MeV ; et
le procédé produit un matériau comprenant des fibres polymères de poly(ester urée) ; et le matériau présente un niveau de garantie de stérilité ayant un niveau de probabilité inférieur à environ 10⁻⁶ après l'étape d'exposition du matériau à l'irradiation.

2. Procédé selon la revendication 1,
dans lequel l'irradiation par faisceau d'électrons présente une énergie d'environ 10 MeV ; et/ou
dans lequel l'étape d'exposition du matériau comprenant les fibres polymères de poly(ester urée) à l'irradiation est effectuée durant plus d'environ 1 min.

3. Procédé selon l'une quelconque des revendications précédentes, dans lequel le polymère de poly(ester urée) présente un poids moléculaire moyen en nombre supérieur à environ 5 000 daltons tel que déterminé par chromatographie d'exclusion après ladite exposition d'un matériau comprenant des fibres polymères de poly(ester urée) à une irradiation.

4. Procédé selon l'une quelconque des revendications précédentes, dans lequel le polymère de poly(ester urée) présente un poids moléculaire moyen en poids supérieur à environ 5 000 daltons tel que déterminé par chromatographie d'exclusion après ladite exposition d'un matériau qui comprend des fibres polymères de poly(ester urée) à une irradiation par un faisceau d'électrons.

5. Procédé selon l'une quelconque des revendications précédentes, dans lequel le polymère de poly(ester urée) présente la structure chimique : 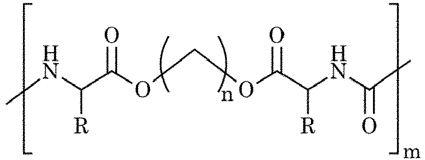 et dans lequel n et m sont des nombres entiers positifs indépendamment sélectionnés supérieurs ou égaux à 1.

6. Procédé selon la revendication 5, dans lequel n est un nombre entier positif compris entre 1 et 20 ; facultativement dans lequel n a la valeur de 6 ou n a la valeur de 10.

7. Procédé selon la revendication 5, dans lequel m est un nombre entier positif compris entre 1 et 1 000.

8. Procédé selon l'une quelconque des revendications 5 à 7, dans lequel R est sélectionné parmi n'importe laquelle des chaînes latérales d'acide aminé d'origine naturelle ; facultativement dans lequel R est sélectionné dans le groupe constitué des substituants isobutyle et benzyle.

9. Procédé selon l'une quelconque des revendications précédentes, dans lequel ladite exposition ne réduit pas le poids moléculaire moyen du polymère de poly(ester urée) de plus de 50 pour cent.

10. Procédé selon l'une quelconque des revendications précédentes, dans lequel ladite exposition ne réduit pas la distribution des poids moléculaires de plus de 50 pour cent dans lequel la distribution des poids moléculaires est calculée en divisant le poids moléculaire moyen en poids par le poids moléculaire moyen en nombre.

11. Procédé selon l'une quelconque des revendications précédentes, dans lequel la différence entre la distribution des poids moléculaires du polymère de poly(ester urée) avant ladite exposition et après ladite exposition est inférieure à environ 50 pour cent, dans lequel la distribution des poids moléculaires est calculée en divisant le poids moléculaire moyen en poids par le poids moléculaire moyen en nombre.

12. Procédé selon l'une quelconque des revendications précédentes, dans lequel le polymère de poly(ester urée) est réticulé avant ladite exposition ; facultativement dans lequel la réticulation avant ladite exposition est effectuée en utilisant un agent de réticulation à base de peptide ; et facultativement en outre dans lequel ledit agent de réticulation à base de peptide présente la structure chimique 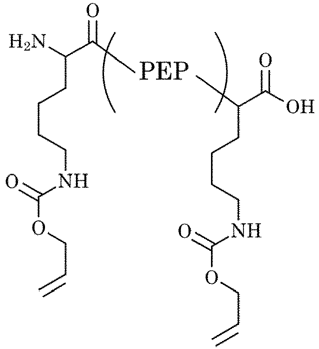 et dans lequel le PEP est une chaîne polypeptidique comprenant de 2 à 20 résidus d'acides aminés.

13. Procédé selon la revendication 12, dans lequel le PEP est un polypeptide sélectionné dans le groupe constitué de SEQ ID n° : 1, SEQ ID n° : 2, SEQ ID n° : 3, et SEQ ID n° : 4.

14. Procédé selon l'une quelconque des revendications précédentes, dans lequel le matériau après ladite exposition présente un module d'élasticité d'au moins environ 5 MPa.

15. Procédé selon l'une quelconque des revendications précédentes, dans lequel lesdites fibres polymères de poly(ester urée) comprennent en outre un deuxième polymère qui n'est pas un polymère de poly(ester urée) ; et/ou
dans lequel lesdites fibres polymères de poly(ester urée) comprennent en outre une composition soluble dans l'eau qui n'est pas un polymère de poly(ester urée) ; et facultativement en outre dans lequel ladite composition soluble dans l'eau qui n'est pas un polymère de poly(ester urée) est du chlorure de sodium ou du saccharose.

16. Procédé selon l'une quelconque des revendications précédentes, dans lequel ledit matériau présente une porosité située dans la plage d'environ 20 pour cent à environ 75 pour cent.
